# EUROPEAN PATENT APPLICATION

(11) **EP 3 158 876 A1**
(43) Date of publication of application: **26.04.2017**
(21) Application number: 15811049.4
(22) Date of filing: 23.06.2015
(51) Int. Cl.: A23L 2/00, A61K 9/08, A61K 31/19, A61K 31/194, A61K 31/375, A61K 33/14, A61P 3/12

(54) **SODIUM-CONTAINING PACKAGED BEVERAGE**

(30) Priority: 23.06.2014 JP 2014128365
(71) Applicant: Suntory Beverage & Food Limited, Tokyo 104-0031 (JP)
(72) Inventor: KOIKE, Rina, Kawasaki-shi Kanagawa 211-0067 (JP); IGARASHI, Masanori, Kawasaki-shi Kanagawa 211-0067 (JP)
(74) Representative: Vossius & Partner Patentanwälte Rechtsanwälte mbB
(86) International application number: PCT/JP2015/067966
(87) International publication number: WO 2015/199053

(57) **Abstract**

An object of the present invention is to provide a beverage that not only allows for rapid replenishment of water, carbohydrate and salt for the purpose of taking measures against heatstroke but also is comfortably drinkable.

It was found that sliminess, bitterness, bad aftertaste and unpleasant finish derived from sodium can be reduced by adding coconut water. It was also found that lauric acid, which is a fatty acid abundant in coconut water, exerts an effect to reduce sliminess, bitterness and other discomforts derived from sodium. The present invention provides a lauric acid-containing beverage which is comfortably drinkable since sliminess, bitterness and other discomforts derived from sodium present in the beverage are reduced.

## Description

### TECHNICAL FIELD

The present invention relates to sodium-containing packaged beverages. More particularly, this invention relates to sodium-containing packaged beverages having lauric acid added thereto.

### BACKGROUND ART

Heatstroke has conventionally occurred frequently during exercise or work at high temperatures, but recently this condition increasingly occurs in daily life due to heat island phenomenon and global warming.

Heatstroke is a condition in which the regulatory functions in the body decline significantly under high temperature environment due to loss of the water-salt balance in the body or insufficient heat dissipation. Depending on the symptoms, this condition is divided into three groups: heat cramps, heat exhaustion, and thermic fever associated with impairment of body's heat regulatory function. In severe cases, heatstroke may result in death. There are various cases of heatstroke, such as cases where the condition occurs in infants or the elderly under heat wave or high temperature environment, where the condition occurs during work under hot environment, and where the condition occurs during exercise. It is known that particularly during exercise, heatstroke can occur even not in high temperature environment, due to various influences including generation of large amounts of heat from the muscle or dehydration from sweating.

With regard to measures against heatstroke, it is reported that a feeling of fatigue after exercise is reduced by ingesting the same amount of water as that lost by sweating, and that increase in core body temperature is suppressed by intake of water on a daily basis. Therefore, appropriate water replacement and suppression of rise in core body temperature are important to prevent heatstroke. Also, since not only water but also salt are lost by sweating, it is necessary to replenish water and salt together particularly during heavy sweating. In addition, it is also required to replenish carbohydrate during onset of heatstroke because this component not only provides a supply of energy but also has the ability to promote salt absorption. That is, appropriate replenishment of three components - - water, salt and carbohydrate -- is essential for prevention of heatstroke.

There are many reports on sport drinks with adjusted osmotic pressure, electrolyte concentration and sugar concentration, which are designed to replenish water and electrolytes lost during exercise or work at high temperatures (*e.g*., Patent Literatures 1 and 2). In particular, Patent Literatures 3 and 4 make mention of sport drinks from the viewpoint of prevention of heatstroke. Further, in the United States, coconut water has been consumed as a drink for replenishing potassium lost through sweating.

The aforementioned sport drinks are provided for the purposes of replenishing water and electrolytes lost by sweating during exercise or strenuous activity and providing a source of energy required for maintaining vital functions of the body. For this reason, electrolytes are abundantly included in those sport drinks, but it has been pointed out that sliminess, bitterness, bad aftertaste, and unpleasant finish caused by electrolytes other than sodium are sensed when one drinks such sport drinks. As a solution for this issue, it was found that sliminess, bitterness and other problems derived from electrolytes are reduced by incorporating electrolytes in the form of natural materials (Patent Literature 5).

### CITATION LIST

### PATENT LITERATURES

Patent Literature 1: Japanese Patent Application Publication No. JP 2004-123642
Patent Literature 2: Japanese Patent Application Publication No. JP 2006-304775
Patent Literature 3: Japanese Patent Application Publication No. JP 2008-072968
Patent Literature 4: Japanese Patent Application Publication No. JP 2010-057408
Patent Literature 5: Japanese Patent Application Publication No. JP 2013-009661

### SUMMARY OF INVENTION

### TECHNICAL PROBLEM

Sport drinks have included therein an abundance of electrolytes and sugars for the purposes of not only replenishing water and electrolytes lost through exercise or strenuous activity but also providing a source of energy. Particularly from the viewpoint of prevention of heatstroke, it is important to appropriately replenish salt lost by sweating, and thus beverages intended to provide measures against heatstroke have included therein a large amount of sodium (*e.g*., sodium chloride (common salt)). However, as a result of the prevent inventors' study, it was found that beverages containing a large amount of sodium give rise to sliminess, bitterness, bad aftertaste and unpleasant finish when the beverages are drunk. The present invention has as its object to provide beverages that not only allow for rapid replenishment of water, salt and carbohydrate but also are comfortably drinkable, by reducing sliminess, bitterness and other discomforts caused by the presence of high concentrations of sodium.

### SOLUTION TO PROBLEM

The present inventors found that lauric acid, which is a fatty acid, exerts an effect to reduce sliminess, bitterness and other discomforts derived from sodium, and thus have completed the present invention.

More specifically, the present invention is directed to, but not limited to, the following.
1) A packaged beverage having a lauric acid content of 0.300 to 2.00 ppm and a sodium content of 130 to 600 ppm.
2) The packaged beverage as set forth in 1), wherein the lauric acid content is in the range of 0.400 to 2.00 ppm.
3) The packaged beverage as set forth in 1) or 2), wherein the sodium content is in the range of 150 to 600 ppm.
4) The packaged beverage as set forth in any of 1) to 3), wherein the packaged beverage has a ratio of lauric acid content to sodium content which is in the range of lauric acid:sodium=1:100 to 1:2000.
5) The packaged beverage as set forth in any of 1) to 4), wherein the lauric acid is incorporated in the form of coconut water.
6) The packaged beverage as set forth in any of 1) to 5), wherein the packaged beverage is a beverage obtained through addition of a sugar and having a Brix value of not more than 12.
7) The packaged beverage as set forth in any of 1) to 6), wherein the packaged beverage is a beverage obtained through addition of an acidulant and having a pH of 2 to 6.
8) A method for producing a beverage, comprising the steps of:
   (i) incorporating lauric acid so as to give a final lauric acid concentration in the beverage of 0.300 to 2.00 ppm,
   (ii) incorporating sodium so as to give a final sodium concentration in the beverage of 130 to 600 ppm,
   (iii) sterilizing the beverage (liquid preparation), and
   (iv) packaging the beverage (liquid preparation).
9) The method as set forth in 8), wherein the final lauric acid concentration in the beverage is in the range of 0.300 to 2.00 ppm.
10) The method as set forth in 8) or 9), wherein the final sodium concentration in the beverage is in the range of 130 to 600 ppm.
11) The method as set forth in any of 8) to 10), wherein the lauric acid is incorporated in the form of coconut water.

### ADVANTAGEOUS EFFECTS OF INVENTION

The beverage of the present invention is characterized in that a specified concentration of lauric acid is incorporated in a beverage containing a large amount of sodium to thereby reduce sliminess, bitterness, bad aftertaste and unpleasant finish caused by sodium. Sliminess, bitterness and other discomforts derived from sodium are also reduced by incorporating coconut water rich in lauric acid. Since the inventive beverage does not make one perceive sliminess, bitterness or any other discomforts derived from sodium, the beverage is comfortably drinkable and allows for easy replenishment of water, salt and carbohydrate during exercise or work at high temperatures.

### DESCRIPTION OF EMBODIMENTS

The present invention relates to beverages comprising lauric acid and sodium.

The present inventors found that although fatty acids *per se* have sliminess, lauric acid, which is a kind of fatty acid, has an effect to mask sliminess, bitterness and other discomforts derived from sodium. The present invention is characterized by using lauric acid, which *per se* has sliminess, as a component for masking the sliminess and bitterness of a beverage containing a large amount of sodium.

As referred to herein, "sliminess" refers to a sensation on the tongue which lingers from the time when a beverage is put into the mouth and after the beverage is swallowedthis is an element that hinders comfortable drinking and gives a bad influence on the aftertaste and finish of a beverage.

### [Constitution of beverage]

The beverage of the present invention is characterized by comprising lauric acid and sodium.

The sodium concentration in the inventive beverage is in the range of 130 to 600 ppm, preferably 150 to 600 ppm, more preferably 200 to 600 ppm. Too low a concentration of sodium is insufficient to replenish water and salt lost by sweating.

The sodium can be from any source as long as it is a drinkable sodium salt; and examples of the sodium that can be used include, but are not limited to, sodium chloride (common salt), disodium citrate, trisodium citrate, sodium L-aspartate, sodium benzoate, and sodium bicarbonate.

Lauric acid is a saturated fatty acid having 12 carbon atoms, and is also called dodecanoic acid. It is known that lauric acid is present in mother's milk and the like, and has immunostimulatory and antimicrobial activities.

The concentration of lauric acid included in the inventive beverage is in the range of 0.300 to 2.00 ppm, preferably 0.400 to 2.00 ppm, more preferably 0.700 to 2.00 ppm. It should be noted that the aforementioned content is a total amount of lauric acid. The content of lauric acid can be measured using any known method such as HPLC.

Herein, "ppm" used as a unit of the concentration of a substance contained in the inventive beverage expresses the weight (mg) of the substance per liter of the beverage, namely mg/L.

The inventive beverage may have coconut water incorporated therein as a source of lauric acid. As referred to herein, coconut water refers to a semitransparent liquid albumen contained in immature coconut fruits. The nutritious juice rich in minerals which has been enjoyed in the life of people in tropical countries is a natural water supply ingredient having an osmotic pressure comparable to that of the body and a mineral constitution close to that of body fluids. Coconut water, which is rich in magnesium, potassium and other minerals, is known to be effective in reducing swelling and activating metabolic enzymes. It is also rich in lauric acid, as found in mother's milk, and has immunostimulatory and other activities.

In the inventive beverage, coconut water is incorporated in such an amount as to give a lauric acid concentration in the beverage of 0.300 to 2.00 ppm, preferably 0.400 to 2.00 ppm, more preferably 0.700 to 2.00 ppm. The concentration of coconut water to be incorporated in the inventive beverage is in the range of preferably 2 to 10% by weight, more preferably 3 to 9% by weight, still more preferably 4 to 8% by weight. Additionally, the amount of lauric acid present in coconut water can be measured using any known method such as HPLC.

The ratio of lauric acid content to sodium content is in the range of lauric acid:sodium=1: 100 to 1:2000, preferably 1:250 to 1:1375, more preferably 1:375 to 1:800.

In the inventive beverage, one or more types of sugars can be incorporated. The type(s) of a sugar(s) is(are) not particularly limited, and examples of sugars include monosaccharides such as glucose (grape sugar), galactose, mannose and fructose (fruit sugar), disaccharides such as maltose, lactose and sucrose (cane sugar), polysaccharides such as oligosaccharides, dextrin, hyaluronic acid and alginic acid, natural sweeteners such as honey, maple syrup, erythritol, trehalose, xylitol, sorbitol and stevia, and artificial sweeteners such as sucralose, aspartame and acesulfame potassium.

The inventive beverage may have added thereto a fruit juice derived from a fruit compatible with the tastes of sodium and lauric acid. One type of fruit juice may be used, or two or more types of fruit juices may be used in combination. The type(s) of a fruit juice(s) is(are) not particularly limited as long as the juice(s) is(are) derived from a fruit(s) commonly used to make juice; and examples of fruits include citrus fruits (*e.g*., grapefruit, white grape, lemon, lime, orange), stone fruits (e.g., apricot, peach, cherry, Japanese apricot, Japanese plum), berries (e.g., muscat, Delaware, Kyoho, Pione), strawberry, apple, pear, kiwi fruit, pineapple, and mango.

The sugar content in the inventive beverage is not particularly limited and can be determined depending on the quality characteristics of the beverage, and the sugar content is preferably not more than 12 Brix, more preferably not more than 10 Brix, still more preferably 4 to 9 Brix. The Brix value is a way to determine the sugar concentration of a beverage based on its refractive index. To be specific, it is a value obtained by converting the refractive index of a test liquid into the percent by weight concentration (w/w%) of a sucrose solution at 20°C. Sugar concentration may be determined using any other known method besides Brix value, such as biosensor method and Bertrant's method.

The inventive beverage may have an acidulant added thereto. Specific examples of the acidulant include citric acid (crystal) (anhydrous), trisodium citrate, DL-malic acid, tartaric acid, lactic acid and phosphoric acid.

The pH of the inventive beverage is not particularly limited and is in the range of preferably 2 to 6, more preferably 2 to 5, still more preferably 3 to 4.

Like common beverages, the inventive beverage may have various additives incorporated therein to the extent that the effects of the present invention are not adversely affected. Examples of additives include, but are not limited to, flavorants, vitamins, pigments, antioxidants, emulsifiers, preservatives, seasonings, extracts, pH adjustors, and quality stabilizers.

### [Type of beverage and container for beverage]

In the present invention, the type of beverage is not particularly limited, and examples include soft drinks such as carbonated beverages, fruit and fruit juice beverages, coffee beverages, tea-based beverages, tea beverages (e.g., green tea, oolong tea, black tea), soy milk, vegetable beverages, sport dinks and milky beverages, as well as dairy beverages and lactobacillus beverages. Especially, it is preferred that the inventive beverage be a sport drink, in particular, a beverage having an osmotic pressure equal to that of bodily fluids of healthy humans (about 280 to 290 mOsm/kg) (*i.e*., isotonic drink) or a beverage having an osmotic pressure lower than that of human bodily fluids (*i.e*., hypotonic drink).

The inventive beverage is advantageously provided as a beverage for prevention of heatstroke. Examples of heatstroke that the inventive beverage is intended to prevent include, but are not limited to, heatstroke occurring in infants or the elderly at high temperatures, heatstroke occurring in workers under heat environment, and heatstroke occurring during exercise. The inventive beverage can also be provided for the purpose of replenishing sodium and carbohydrate. For example, the inventive beverage is provided for various purposes including, but not limited to, recovery from dehydration, water and nutritional support, and prevention of heatstroke.

The inventive beverage can be made into a packaged beverage. The container to be used for the packaged beverage is not particularly limited, and any containers commonly used for beverages can be used, including resin containers such as PET bottle, paper containers such as paper pack, glass containers such as glass bottle, metal containers such as aluminum can and steel can, and aluminum pouch.

### [Method for producing beverage]

In another aspect, the present invention is directed to a method for producing a beverage. The inventive method includes the steps of incorporating lauric acid and sodium in a beverage. Sodium is incorporated so as to give a final sodium concentration in the beverage of 130 to 600 ppm, preferably 150 to 600 ppm, more preferably 200 to 600 ppm. Lauric acid is incorporated so as to give a final lauric acid concentration in the beverage of 0.300 to 2.00 ppm, preferably 0.400 to 2.00 ppm, more preferably 0.700 to 2.00 ppm. The intended concentrations of sodium and lauric acid can be achieved through, for example, adjustment of their amounts to be added at the incorporation steps. The forms in which sodium and lauric acid are incorporated and the methods for incorporating these ingredients are not particularly limited; and sodium and lauric acid can be added as source ingredients, either *per se* or in the form of an article(s) containing them, at any given timing in the production process.

Also, the present invention is directed to a method for producing a beverage which includes the step of incorporating lauric acid in the form of coconut water. In this inventive method, the amount of coconut water to be added is adjusted at the incorporation step such that the final concentration of lauric acid in a beverage is adjusted to be in the range of 0.300 to 2.00 ppm, preferably 0.400 to 2.00 ppm, more preferably 0.500 to 2.00 ppm. That is, coconut water is incorporated while the final concentration of coconut water in the beverage is adjusted to be in the range of preferably 2 to 10% by weight, more preferably 3 to 9% by weight, still more preferably 4 to 8% by weight. The form in which coconut water is incorporated and the method for incorporating coconut water are not particularly limited; and coconut water can be added as a source ingredient at any given timing in the production process.

As the need arises, further steps can be added as appropriate, including sugar incorporation step, osmotic pressure adjustment step, pH adjustment step, additive inclusion step, sterilization step, and packaging step. In a preferred mode, the inventive beverage can be made into a packaged beverage, or a sterilized, packaged beverage. For example, the sterilized, packaged beverage can be produced by packing a liquid preparation comprising lauric acid and sodium in a container and then subjecting the liquid preparation to heat sterilization such as retort sterilization, or by sterilizing such a liquid preparation before packing it in a container. To put it more specifically, in the case of producing a beverage packed in a metal container such as a can, the liquid preparation of the present invention packed in such a container in a specified amount can be subjected to a sterilization step. In the cases of producing a PET bottle beverage, a paper packed beverage, a bottled beverage, a canned beverage, or a pouched beverage, ultraheat treatment can be carried out, for example, at 90 to 130°C for a few to several tens of seconds. When the inventive beverage is made into a packaged beverage, hot pack filling or aseptic packaging can be employed.

### EXAMPLES

Hereunder, the present invention will be specifically described by way of working examples, but this invention is not limited to these examples. Unless otherwise specified, all numerical ranges given herein include their endpoints.

### Example 1

### [Table 1]

**Table 1.**

| | | |
|---|---|---|
| High fructose corn syrup | 85.0 | g |
| Fructose | 15.0 | 9 |
| Citric acid (anhydrous) | 1.00 | g |
| Phosphoric acid 75% | 0.900 | g |
| Sodium ascorbate | 0.150 | g |
| Trisodium citrate | 1.20 | g |
| Common salt | 0.300 | g |
| | 1000 | mL |

Liquid preparations were prepared by the following procedure: different source ingredients were suitably mixed in the relative proportion shown in the above table, then, lauric acid was added to the mixture at varied concentrations ranging from 0 to 0.4 ppm, and the resulting liquid preparations were sterilized at 95°C for 30 seconds and packed in 500 mL PET bottles.

The prepared beverages were subjected to sensory evaluation by three specialized panelists.

The sensory evaluation was conducted in terms of improvement in bitterness, harshness and sliminess derived from minerals, using a 5-point rating scale (5: remarkably improved; 4: fairly improved; 3: improved; 2: slightly improved; and 1: not improved at all), and the rated scores were averaged. 3 or higher points were regarded as working well. The sensory evaluation was performed on both the beverages cooled to below 10°C and the beverages kept at room temperature.

Table 2 shows the results of the sensory evaluation performed on the cooled beverages. The results revealed that it is effective to add lauric acid, but that a beverage having lauric acid added thereto in an amount exceeding a certain level is not suitable for use as a drink because oil floating occurs in the beverage.

### [Table 2]

**Table 2.**

| | | | | | | | |
|---|---|---|---|---|---|---|---|
| Sodium (ppm) | | 400 | | | | | |
| Lauric acid (ppm) | | 0 | 0.1 | 0.3 | 0.7 | 2.0 | 4.0 |
| Ratio | | -- | 1:4000 | 1:1333 | 1:570 | 1:200 | 1:100 |
| Sensory evaluation results | Bitterness/ harshness | 1 | 2 | 4 | 5 | 5 | Not suitable |
| | Sliminess | 1 | 1 | 3 | 4 | 5 | Not suitable |

### Example 2

A beverage composition was prepared as in Example 1, and each of lauric acid and other edible oils and fat was added at a concentration of 0.7 ppm to the beverage composition, and the resulting liquid preparations were sterilized at 95°C for 30 seconds and packed in 500 mL PET bottles. The prepared beverages were subjected to sensory evaluation in the same manner as in Example 1.

### [Table 3]

**Table 3.**

| | Lauric acid | Medium chain triglyceride | Corn oil | Olive oil | Rapeseed oil |
|---|---|---|---|---|---|
| Sensory evaluation results | 5 | 2 | -- | | |
| | | | (Unable to be evaluated because of being unsuitable for formulation) | | |

Medium chain triglyceride did not take full effect. The beverages having added thereto corn oil, olive oil, or rapeseed oil were unable to be subjected to sensory evaluation because they were significantly unsuitable for being formulated into preparations.

### Example 3

### [Table 4]

**Table 4.**

| | | |
|---|---|---|
| High fructose corn syrup | 85.0 | g |
| Fructose | 15.0 | g |
| Citric acid (anhydrous) | 1.00 | g |
| Phosphoric acid 75% | 0.900 | g |
| Lauric acid | 0.700 | mg |
| | 1000 | mL |

Beverage compositions were prepared by the following procedure: different source ingredients were suitably mixed in the relative proportion shown in the above table, then, sodium was added to the mixture at varied concentrations ranging from 0 to 80 mg/100 mL, and the resulting liquid preparations were sterilized at 95°C for 30 seconds and packed in 500 mL PET bottles.

The prepared beverages were subjected to sensory evaluation by three specialized panelists.

The sensory evaluation was conducted in terms of suitability for use as a functional beverage, using the following 5-point rating scale: 5 (favorable), 4 (somewhat favorable), 3 (neither favorable nor unfavorable), 2 (somewhat unfavorable), and 1 (unfavorable). The rated scores were averaged.

Table 5 shows the results of the sensory evaluation performed on the cooled beverages. It was found that a beverage containing 13 mg or more of sodium has a favorable flavor suitable for a functional beverage, but that a beverage containing 80 mg or more of sodium tastes salty and is poorly suitable for use as a drink.

### [Table 5]

**Table 5.**

| | | | | | | | | |
|---|---|---|---|---|---|---|---|---|
| Sodium (ppm) | 0.7 | | | | | | | |
| Lauric acid (ppm) | 0 | 100 | 130 | 150 | 200 | 400 | 600 | 800 |
| Ratio | -- | 1:142 | 1:186 | 1:214 | 1:285 | 1:571 | 1:857 | 1:1143 |
| Sensory evaluation results | 1 | 2 | 4 | 5 | 5 | 5 | 5 | 2 |

### INDUSTRIAL APPLICABILITY

The packaged beverage of the present invention is a beverage designed to allow for easy replenishment of water, salt and carbohydrate, which is improved in sliminess, bitterness, bad aftertaste, and unpleasant finish caused by sodium contained in the beverage at high concentrations. This invention can provide beverages that can be consumed comfortably, though containing salt, for the purpose of taking measures against heatstroke occurring during exercise or work at high temperatures.

## Claims

1. A packaged beverage having a lauric acid content of 0.300 to 2.00 ppm and a sodium content of 130 to 600 ppm.

2. The packaged beverage according to claim 1, wherein the packaged beverage has a ratio of lauric acid content to sodium content which is in the range of lauric acid:sodium=1:100 to 1:2000.

3. The packaged beverage according to any one of claims 1 and 2, wherein the lauric acid is incorporated in the form of coconut water.

4. The packaged beverage according to any one of claims 1 to 3, wherein the packaged beverage has a sugar content of not more than 12 Brix.

5. The packaged beverage according to any one of claims 1 to 4, wherein the packaged beverage is sterilized.
